# EUROPÄISCHE PATENTSCHRIFT

(11) **EP 0 931 838 B1**
(45) Veröffentlichungstag und Bekanntmachung des Hinweises auf die Patenterteilung: **13.02.2002**
(21) Anmeldenummer: 98124397.5
(22) Anmeldetag: 22.12.1998
(51) Int. Cl.: C12P 13/20

(54) **Verfahren zur Herstellung von L-Asparaginsäure**
Process for the production of L-aspartic acid
Procédé de préparation de l'acide aspartique

(30) Priorität: 29.12.1997 AT 219197
(43) Veröffentlichungstag der Anmeldung: 28.07.1999
(73) Patentinhaber: DSM Fine Chemicals Austria Nfg GmbH & Co KG, 4021 Linz (AT)
(72) Erfinder: Giselbrecht, Karl-Heinz, Dr., 4061 Pasching (AT); Schaller, Josef, 4020 Linz (AT)
(74) Vertreter: Klostermann, Ingrid

(56) Entgegenhaltungen:
- EP-A- 0 752 476
- US-A- 4 560 653
- DATABASE WPI Derwent Publications Ltd., London, GB; AN 1996-145947 XP002116572 & JP 08 033493 A (NIPPON SHOKUBAI CO LTD), 6. Februar 1996 (1996-02-06)

## Beschreibung

L-Asparaginsäure ist ein essentieller Ausgangsstoff für verschiedenste Additive für die pharmazeutische Industrie und den Nahrungsmittelsektor. Beispielsweise wird L-Asparaginsäure zur Herstellung von künstlichem Süßstoff, beispielsweise Aspartam, eingesetzt. Zur Herstellung von L-Asparaginsäure sind deshalb bereits eine Vielzahl von chemischen und enzymatischen Verfahren beschrieben. Bei den enzymatischen Varianten wird L-Asparaginsäure zumeist durch enzymatische Addition von Ammoniak an Fumarsäure mit nachfolgender Ausfällung aus der so erhaltenen L-Ammoniumaspartat-Lösung gewonnen.
Das Ausfällen von L-Asparaginsäure kann beispielsweise durch Zugabe einer Mineralsäure wie etwa Schwefelsäure oder Salzsäure oder anderer Säuren wie etwa p-Toluolsulfonsäure erfolgen.
Die Nachteile dabei sind jedoch, daß der Verlust an Ammoniak groß ist und eine große Menge an Abwasser mit einer hohen Konzentration an Ammoniumsalzen der verwendeten Säuren ausgeschieden wird.
Aus diesem Grund wurde versucht, Möglichkeiten zu finden, die Abwasserproblematik zu verringern oder gänzlich zu vermeiden.

Gemäß US 4,560,653 erfolgt das Ausfällen der L-Asparaginsäure beispielsweise durch Zugabe von Maleinsäure. Nach der Abtrennung von L-Asparaginsäure wird die verbleibende Mutterlauge einem Isomerisierungsschritt unterworfen, bei dem Maleinsäure in Fumarsäure etwa mittels eines Bromionen enthaltenden Katalysators isomerisiert wird, anschließend gereinigt und der enzymatischen Reaktion erneut zugeführt wird.

Um den Isomerisierungsschritt zu umgehen, wurde nach weiteren geeigneten Zusätzen zur Ausfällung von L-Asparaginsäure gesucht.
In der japanischen Offenlegungsschrift JP 08-33493 (Chem. Abstracts 1224: 315 167) wird die Verwendung von Fumarsäure bzw. Fumarsäuresalz als Fällungsmittel beschrieben. Der Nachteil dieser Verfahrensvariante liegt in der schlechten Wasserlöslichkeit von Fumarsäure, wodurch bei der Aufarbeitung der Mutterlauge entweder große Mengen an Wasser abdestilliert werden müssen oder eine sehr verdünnte Fahrweise mit großen Reaktionsvolumen erforderlich ist.

Aufgabe der Erfindung war es demnach ein Verfahren zu finden, das die bisherigen Probleme bei der Ausfällung von L-Asparaginsäure vermeidet und das zu L-Asparaginsäure in hohen Ausbeuten und hoher Reinheit führt.

Unerwarteterweise konnte diese Aufgabe durch die Verwendung von Ameisensäure oder Essigsäure als Fällungsmittel und im Kreisfahren aller Reaktionsströme gelöst werden.

Gegenstand der Erfindung ist demnach ein verbessertes Verfahren zur Herstellung von L-Asparaginsäure durch enzymkatalysierte Umsetzung von Fumarsäure mit Ammoniak, das dadurch gekennzeichnet ist, daß
a) Fumarsäure in einem inerten Verdünnungsmittel in Gegenwart von Aspartase oder Aspartase-produzierenden Mikroorganismen mit Ammoniak zu Ammonium-L-Aspartat umgesetzt, anschließend
b) Ammoniak aus dem Reaktionsgemisch entfernt und
c) aus dem verbleibenden Reaktionsgemisch L-Asparaginsäure durch Zugabe von Ameisensäure oder Essigsäure ausgefällt, dann abgetrennt, gewaschen und getrocknet wird, sowie
d) aus der mit dem Waschwasser vereinigten, Ammoniumformiat bzw. -acetat hältigen Mutterlauge unter Zugabe von Fumarsäure Ameisensäure bzw. Essigsäure freigesetzt,
e) nach Filtration der so erhaltenen Ammoniumfumarat hältigen Lösung das Filtrat gegebenenfalls zusammen mit frischer Ameisensäure bzw. Essigsäure wiederum in Schritt c) zur Ausfällung von L-Asparaginsäure und
f) das verbleibende Ammoniumfumarat mit dem Ammoniak aus Schritt b) vermengt und als Edukt in Schritt a) zugesetzt wird.

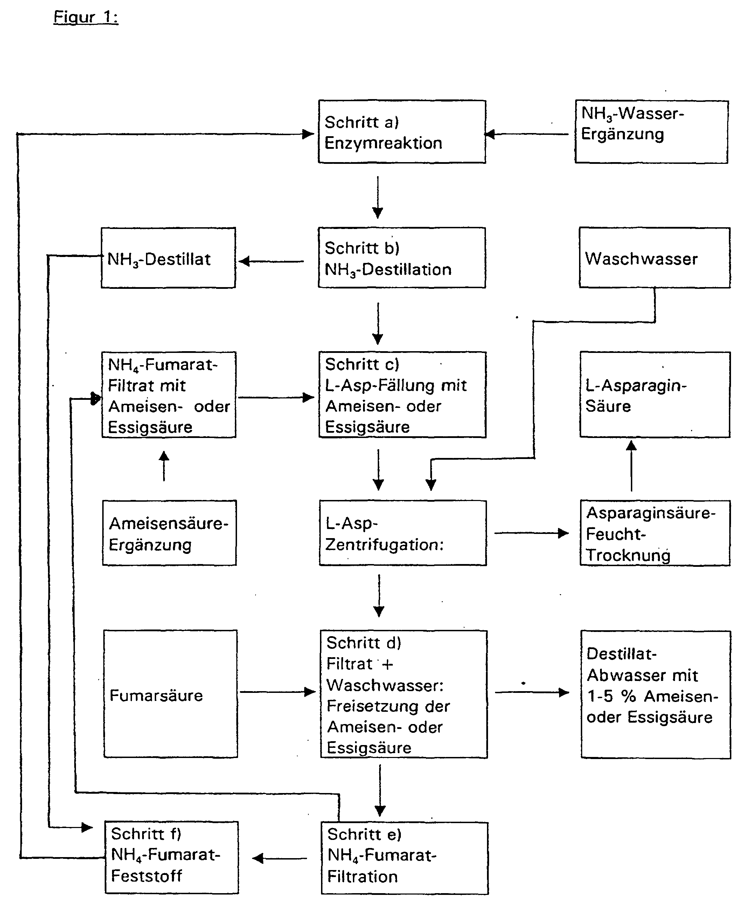

Bei dem erfindungsgemäßen Verfahren wird L-Asparaginsäure mittels Ameisensäure oder Essigsäure ausgefällt. Die Reaktionsströme werden erfindungsgemäß alle im Kreis gefahren. Das erfindungsgemäße Verfahren ist aus Abbildung 1 ersichtlich, in der die Reaktionsströme schematisch dargestellt sind.

Der erste Schritt des erfindungsgemäßen Verfahrens beinhaltet die enzymatische Reaktion der Fumarsäure mit Ammoniak. Die Reaktion findet dabei in einem inerten Verdünnungsmittel statt. Als inerte Verdünnungsmittel eignen sich Wasser, Wasser/Ethanol- oder Wasser/Aceton-Gemische und dergleichen. Bevorzugt wird Wasser eingesetzt. Fumarsäure kann dabei in einer Konzentration bis zur Löslichkeitsgrenze verwendet werden, sodaß entweder eine Lösung oder aber eine Suspension erhalten wird. In diese Lösung bzw. Suspension wird Ammoniak gasförmig, verflüssigt oder in Form einer 10 bis 35 Gew.%igen Lösung eingeleitet, wodurch sich die Temperatur bis zu 60°C erhöht und sich ein pH-Wert zwischen 7 und 9 einstellt.

Bevorzugt wird eine wässrige 20 bis 30 Gew.%ige Ammoniaklösung verwendet. In das so erhaltene System, vorzugsweise eine Lösung, wird sodann bei 20 bis 60°C, bevorzugt bei 30 bis 50°C das Enzym Aspartase oder ein Aspartase-produzierender Mikroorganismus, eingerührt. Bei dieser Zugabe an Enzym- bzw. Aspartase-produzierenden Mikroorganismus ist es von Vorteil, wenn durch die Ammoniakzugabe eine Lösung erhalten wird, da im Falle einer Suspension durch Adsorption des Enzyms und dadurch bedingten Aktivitätsverlust mehr Enzym erforderlich ist. Für einen fast quantitativen Umsatz nach bis zu 24 bis 30 Stunden sind dabei 30 bis 50 IU (Enzymaktivität) pro Mol Fumarsäure erforderlich.
Aspartase-produzierende Mikroorganismen sind beispielsweise Pseudomonas fluorescens, Protens vulgaris, Pseudomonas aeruginosa, Serratia marcescens, Bacterium succinium, Bacillus subtilis, Aerobacter aerogenes, Micrococcus sp. Escherichia coli u. a.
Weitere geeignete Aspartase-produzierende Mikroorganismen sind beispielsweise in US 3,791,926 und US 3,198,712 beschrieben.
Bei dem erfindungsgemäßen Verfahren kann weiters gereinigte oder synthetische Aspartase eingesetzt werden. Das Enzym bzw. der Aspartase-produzierende Mikroorganismus kann in flüssiger oder in immobilisierter Form wie beispielsweise in EP 0 127 940 beschrieben, zugesetzt werden.
Nach vollendeter Reaktion, das Reaktionsende kann beispielsweise photometrisch ermittelt werden, erfolgt gemäß Schritt b) das Abtrennen von Ammoniak mittels Destillation oder Strippen.

Schritt b) kann bei Normaldruck oder bei reduziertem Druck und bei Temperaturen von 30 bis 110°C, bevorzugt von 40 bis 90°C erfolgen.
Überschüssiges Ammoniak wird dabei mittels geläufiger Destillationsmethoden, beispielsweise mittels Kurzweg- oder Dünnschichtverdampfer, Stripper u.s.w., aus dem Reaktionsgemisch entfernt. Je nach Destillationsmethode wird dabei entweder bevorzugt bei Normaldruck oder bei reduziertem Druck zwischen 80 und 200 mbar gearbeitet. Das so erhaltene Ammoniakdestillat wird in einem nachfolgenden Verfahrensschritt, Schritt f), bzw. als Edukt für eine nachfolgende weitere Enzymreaktion a) wiedereingesetzt.
Nach der Ammoniakentfernung erfolgt in Schritt c) das Ausfällen der L-Asparaginsäure.
Dazu wird der durch das Entfernen des Ammoniaks erhaltenen Monoammonium-L-Aspartat-Lösung Ameisensäure oder Essigsäure in einem molaren Verhältnis von Monoammonium-L-Aspartat zu Ameisensäure bzw. Essigsäure gleich 1 : 0,8 bis 2 bevorzugt 1 : 1 bis 1,2 zugesetzt. Bevorzugt wird Ameisensäure als Fällungsmittel eingesetzt. Der pH-Wert bei der Fällung liegt bevorzugt zwischen 4 und 6.

Ab dem 2ten Reaktionszyklus wird Ammoniumfumaratfiltrat, das freigesetzte Ameisensäure bzw. Essigsäure enthält, (erhalten aus dem nachfolgenden Schritt e)) der Monoammonium-L-Aspartat-Lösung als Fällungsmittel zugegeben. Um das gewünschte Molverhältnis zwischen Aspartat und Fällungsmittel zu erreichen, wird das Filtrat gegebenenfalls mit Ameisensäure bzw. Essigsäure ergänzt.
Die Reaktionstemperatur liegt zwischen 0 und 95°C, bevorzugt zwischen 20 und 90°C besonders bevorzugt zwischen 40 und 70°C. Die auskristallisierte L-Asparaginsäure wird sodann aus dem Reaktionsgemisch abfiltriert, beispielsweise durch Absorptionsfiltration oder Zentrifugieren. Bevorzugt wird L-Asparaginsäure durch Zentrifugation abgetrennt. Die Filtriertemperatur liegt zwischen 10 und 70°C, bevorzugt zwischen 15 und 50°C. Die abfiltrierten Kristalle der L-Asparaginsäure werden abschließend gewaschen, vorzugsweise mit Wasser, und getrocknet. Aufgrund der sehr guten Löslichkeit von Ammoniakformiat bzw. - acetat ist für das Waschen der frisch gefällten L-Asparaginsäure nur sehr wenig Waschwasser notwendig. L-Asparaginsäure kann somit auch bei geringeren Temperaturen gewaschen werden. Enthält L-Asparaginsäure Ammoniumfumarat durch die Verwendung von Fumarsäure als Fällungsmittel, sind hingegen höhere Waschtemperaturen und Waschwassermengen notwendig.
Das verbleibende, nach Abtrennung von L-Asparaginsäure erhaltene Filtrat, sowie das beim Waschen anfallende Waschwasser werden vereinigt, gegebenenfalls bis zur Hälfte eingedampft, mit Fumarsäure versetzt und zur Wiederfreisetzung der Ameisensäure bzw. Essigsäure, in Abhängigkeit von der gewählten Destillationsmethode unter reduziertem Druck oder Normaldruck bis auf Siedetemperatur erhitzt.
Bevorzugt erfolgt die Zugabe der Fumarsäure bei max. 100°C, besonders bevorzugt bei max. 70°C. Destillatwasser wird dabei gemeinsam mit einem kleinen Teil, etwa mit 1 bis 5 Mol% der Ameisensäure bzw. Essigsäure abdestilliert.
Die verbleibende Lösung wird filtriert bzw. zentrifugiert und das den Großteil der Ameisensäure bzw. Essigsäure enthaltende Ammonium-Fumarat-Filtrat wird ab dem 2ten Zyklus in Schritt c) zur Ausfällung von L-Asparaginsäure wiedereingesetzt.
Dazu wird gegebenenfalls die bei der Freisetzung der Säure abdestillierte Menge durch Zugabe von frischer Ameisensäure bzw. Essigsäure ergänzt.
Der verbleibende Ammonium-Fumarat-Feststoff wird hingegen mit dem in Schritt b) abdestillierten Ammoniak vereinigt und ab dem 2ten Zyklus, der enzymkatalysierten Reaktion als Edukt in Schritt a) zugesetzt. Gleichzeitig wird die fehlende Ammoniakmenge durch Zugabe von Ammoniak, bevorzugt in Form einer 10 bis 35 Gew.%igen Lösung, ergänzt, sodaß wiederum ein pH-Wert zwischen 7 und 9 eingestellt wird.
Eventuell vorhandenes Ammoniumformiat stört die Enzymreaktion nicht. Durch das erfindungsgemäße Verfahren wird L-Asparaginsäure in einer Ausbeute von über 80%, bis zu 95 % und einem Gehalt von über 99,5 % erhalten.

### Beispiel 1:

Von 900 ml (955 g) L-Asparaginsäure-Rekationslösung, (DSM-Chemie Linz) enhaltend 240,6 g (1,80 mol) L-Asparaginsäure, hergestellt durch Reaktion aus 210 g (1,8 mol) Fumarsäure 406 ml 25 Gew.%ige Ammoniak-Lösung (366 g) in 420 ml H₂O in Gegenwart von 0,15 ml Aspartase-Lösung (1000 IU/ml), wurden am Rotavapor bei 75°C und 150 mbar 526 g NH₄OH abdestilliert. Der Sumpf wurde auf 955 g mit dest. H₂O verdünnt, Enzymreste abgesaugt und 82,8 g (1,8 mol) Ameisensäure bei 85°C zugegeben. (pH 5,5) Nach 15 Minuten wurde der ausgefallene Niederschlag bei 85°C von der überstehenden Lösung abgetrennt. Es wurden 251,9 g Feststoff (F1) und 744 g Mutterlauge 1 (Mula 1) erhalten. Die Mutterlauge (Mula 1) wurde auf Raumtemperatur abgekühlt, und von dem dabei ausfallenden Feststoff abgetrennt.
Dabei wurden 51,7 g Feststoff (F 1.1) und 678,5 g Mutterlauge (Mula 1.1) erhalten.
Die beiden Feststoffmengen wurden vereinigt, mit.250 ml H₂O dest. vereinigt, auf Rückfluß erhitzt auf Raumtemperatur abgekühlt und der ausgefallene Feststoff von der Mutterlauge abgetrennt.

| | |
|---|---|
| Mula 2 | 191 g |
| F2 | 288,0 g (mit 30,1 % H₂O) |

F2 wurde wiederum mit 250 ml H₂O dest. versetzt, erneut auf Rückflußtemperatur erhitzt und anschließend auf Raumtemperatur abgekühlt.

| | |
|---|---|
| Mula 3 | 244 g |
| F3 | 286,3 (mit 33,4 % H₂O) |

F3 wurde bei 70°C und Vollvakuum getrocknet, wodurch 195,2 g (= 81,5 % d. Th.) L-Asparaginsäure (mit 0,1 % H₂O) erhalten wurden.

Zur Aufarbeitung der Mutterlaugen wurden sämtliche Chargen (Mula 1, 1.1,2 und 3) vereint (1130 g, 1060 ml) und am Rotavapor bei 70°C und ca 150 mbar 525 ml H₂O abdestilliert. Der Sumpf (565 g) wurde mit 210 g ( 1,8 mol) Fumarsäure versetzt und auf Siedetemperatur (102°C) erhitzt, wodurch sämtlicher Feststoff gelöst wurde.
Anschließend wurde eine Vakuumdestillation bei 100 mbar und einem Rücklaufverhältnis von 5 : 1 durchgeführt.

| | | |
|---|---|---|
| Destillat 1 | 100 g Sumpftemperatur | 48,5 °C |
| | Kopftemperatur | 46,8 °C |
| Destillat 2 | 102 g Sumpftemperatur | 48,5 °C |
| | Kopftemperatur | 48,8 °C |

Bei totaler Abnahme wurde weiterdestilliert.
Es wurden 1823 g Destillat insgesamt erhalten.
Der verbleibende Sumpf wurde auf Raumtemperatur abgekühlt und der ausgefallene Feststoff abgesauft.

| | |
|---|---|
| Mula 4 | 543,8 g |
| F4 | 360 g (18 % H₂O) |

F4 (entspricht 2,1 mol Fumarsäure) wurden sodann in 441 g H₂O dest. gelöst, mit 353,5 g (2,1 mol) Ammoniak 25 %ig, (pH 9,3) versetzt und in Gegenwart von 0,15 g Aspartase 24 Stunden bei 50°C im Trockenschrank reagieren gelassen.
Dabei wurde ein Umsatz von 99,1 % erzielt.

### Beispiel 2:

### Reaktionszyklus 1:

Der 1. Zyklus wurde analog Beispiel 1 durgeführt.
Es wurden wiederum 195 g L-Asparaginsäure (0,1 % H₂O) erhalten (81,5 % d. Th.)
Der Gehalt an Ameisensäure betrug 0,1 %, der Gehalt an Fumarsäure 0,3 % zur Aufarbeitung der Mutterlaugen wurden wiederum alle Chargen vereint und bei 70°C und ca. 150 mbar 525 g abdestilliert.
565 g Sumpf wurden wiederum mit 210 g (1,8 mol) Fumarsäure versetzt auf Siedetemperatur (102°C) erhitzt, auf 10°C gekühlt und der ausgefallene Niederschlag abgetrennt.
369 g Mula 4 wurden erhalten. Der Feststoff (F4) wurde mit 100 ml H₂O dest. auf der Nutsche nachgewaschen.

| | |
|---|---|
| F4 | 367,8 g (32,6 % H₂O) |
| Waschwasser (W4) | 115 g |

### Reaktionszyklus 2:

367,8 g F4 wurden mit 450 g H₂O dest und 238,2 g (3,4 mol) Ammoniakwasser 25 %ig versetzt und in Gegenwart von 0,13 g Aspartase bei pH 8,7 und 50°C 72 Stunden im Trockenschrank reagieren gelassen.
Anschließend wurde die so erhaltene Reaktionslösung (1050 g, 945 ml) bei 70°C und ca. 150 mbar etwa zur Hälfte eingeengt.
587,5 g Destillat
450 g Sumpf (Enzymreste wurden abfiltriert, pH 5,4).
Der Sumpf wurde auf 80°C erwärmt und so rasch als möglich mit Mischung aus Mula 4 und W4 aus Reaktionszyklus 1 und 14 g (17 %) Ameisensäure frisch zugegeben. (pH 5,3).

Dabei wurde auf 10 °C gekühlt. Anschließend wurde der ausgefallene Feststoff (F5) abzentrifugiert und mit 200 ml H₂O (W5) nachgewaschen.

| | |
|---|---|
| Mula 5 | 606 g |
| W5 | 203 g |
| F5 | 262,4 g (40,4 % H₂O) |

F5 wurde bei 75°C und im Vakuum getrocknet.
Es wurden 154,2 g (64,6 % d. Th.) L-Asparaginsäure erhalten (Gehalt Ameisensäure 0,1 %, Gehalt Fumarsäure 0,3 %).
Mula 5 und W5 (791 g) wurden vereinigt und bei 70°C und ca. 150 mbar eingeengt.
Der verbleibende Sumpf (500 g) wurde auf 80°C erhitzt und langsam mit 210 g (1,8 mol) Fumarsäure versetzt, auf Rückfluß erhitzt (105 °C) mit 300 ml H₂O dest. verdünnt, sodaß eine Lösung entstand (T 101°C) und anschließend auf Raumtemperatur abgekühlt. Der ausgefallene Feststoff (F6) wurde abzentrifugiert und gewaschen.

| | |
|---|---|
| Mula 6 | 634 g |
| W6 | 104 g |
| F6 | 296g, (12,3 % H₂O) |

### Reaktionszyklus 3:

F6 aus Reaktionszyklus 2 wurde mit 587 g Ammoniakdestillat aus Reaktionszyklus 2 und 177 g (2,52 mol) Ammoniak frisch versetzt und in Gegenwart von 0,2 ml Enzym bei 50°C 24 Stunden im Trockenschrank reagieren gelassen. (98,4 % Umsatz). Die so erhaltene Reaktionslösung (1015 g) wurde bei 70°C und ca 150 mbar etwa zur Hälfte eingeengt. (Destillat: 481 g)
Der verbleibende Sumpf (526 g) wurde nach Abfiltrieren von Enzymresten, auf 65°C erwärmt und langsam mit 738 g einer Mischung aus Mula 6 und W6 versetzt.
Anschließend wurde 2 Stunden bei 65°C gerührt, auf 10°C gekühlt, der ausgefallene Feststoff (F7) abzentrifugiert und mit 200 ml H₂O (W7) nachgewaschen.

| | |
|---|---|
| Mula 7 | 1019 g |
| W7 | 200 g |
| F7 | 168 g (7,7 % H₂O) |

F7 wurde bei 75°C im Vakuum getrocknet.
Es wurden 154,2 g (64,4 % d.Th.) L-Asparaginsäure erhalten.

Mula 7 und W7 wurden vereinigt und bei 70°C und 150 mbar eingeengt. 504,6 g Sumpf wurden sodann auf 70°C erwärmt mit 210 g (1,8 mol) fumarsäure und 100 ml H₂O versetzt, 2 h gerührt, auf Raumtemperatur gekühlt und der ausgefallene Feststoff (F8) abzentrifugiert und gewaschen (W8)

| | |
|---|---|
| Mula 8 | 337 g |
| W8 | 196,4 g |
| F8 | 404g (18,5 % H₂O) |

### Reaktionszyklus 4:

404 g F8 wurde mit 481 g Ammoniakdestillat aus Zyklus 3 und 177 g frischem Ammoniak versetzt und mit 0,2 ml Enzym bei pH 8,7 und 50°C 24 h reagieren gelassen (98,7 % Umsatz).
Die Aufarbeitungsschritte erfolgten analog den Zyklen 1 - 3.

Es wurden 315 g (131,8 % d. Th) an L-Asparaginsäure erhalten.
Abschließend wurde analog den Reaktionszyklen 1 - 4 ein 5ter Zyklus durchgeführt.
Die Mengenbilanz der 5 Zyklen ist aus Tabelle 1 ersichtlich.

**Tabelle 1**

| | FS (mol) | NH₄-FS (mol) | NH₃ (mol) | AS (mol) | L-Asp. (mol) | L-Asp. (Ausbeute%) | pH-L-Asp.-Fällung |
|---|---|---|---|---|---|---|---|
| 1.Zyklus: | 1,80 | 1,80 | 4,32 | 1,80 | 1,46 | 81,50 | 5,5 |
| 2.Zyklus: | 1,80 | 1,85 | 3,40 | 0,30 | 1,15 | 64,40 | 5,3 |
| 3.Zyklus: | 1,80 | 1,95 | 2,50 | 0,00 | 1,10 | 62,60 | 5,0 |
| 4.Zyklus: | 1,80 | 2,47 | 3,00 | 1,80 | 2,36 | 131,80 | 4,4 |
| 5.Zyklus: | 1,80 | 1,84 | 3,00 | 0,00 | 1,81 | 100,80 | 4,4 |
| Summe | 9,00 | 9,91 | 16,22 | 3,90 | 7,89 | 88,58 | |
| FS: Fumarsäure | | | | | | | |
| NH₄FS: Ammoniumfumarat | | | | | | | |
| AS: Ameisensäure | | | | | | | |
| L-Asp: L-Asparaginsäure | | | | | | | |

## Patentansprüche

1. Verfahren zur Herstellung von L-Asparaginsäure durch enzymkatalysierte Umsetzung von Fumarsäure mit Ammoniak, **dadurch gekennzeichnet, daß**
a) Fumarsäure in einem inerten Verdünnungsmittel in Gegenwart von Aspartase oder Aspartase-produzierenden Mikroorganismen mit Ammoniak zu Ammonium-L-Aspartat umgesetzt, anschließend
b) Ammoniak aus dem Reaktionsgemisch entfernt und
c) aus dem verbleibenden Reaktionsgemisch L-Asparaginsäure durch Zugabe von Ameisensäure oder Essigsäure ausgefällt, dann abgetrennt, gewaschen und getrocknet wird, sowie
d) aus der mit dem Waschwasser vereinigten, Ammoniumformiat bzw. acetat hältigen Mutterlauge unter Zugabe von Fumarsäure Ameisensäure bzw. Essigsäure freigesetzt,
e) nach Filtration der so erhaltenen Ammoniumfumarat hältigen Lösung das Filtrat gegebenenfalls zusammen mit frischer Ameisensäure bzw. Essigsäure wiederum in Schritt c) zur Ausfällung von L-Asparaginsäure verwendet und
f) das verbleibende Ammoniumfumarat mit dem Ammoniak aus Schritt b) vermengt und als Edukt in Schritt a) zugesetzt wird.

2. Verfahren nach Anspruch 1, **dadurch gekennzeichnet, daß** als Verdünnungsmittel in Schritt a) Wasser, Wasser/Ethanol- oder Wasser/Aceton-Gemisch verwendet werden.

3. Verfahren nach Anspruch 1, **dadurch gekennzeichnet, daß** in Schritt a) bei einem pH-Wert zwischen 7 und 9 durchgeführt wird.

4. Verfahren nach Anspruch 1, **dadurch gekennzeichnet, daß** Schritt b) bei Temperaturen zwischen 30 und 110°C und bei Normaldruck oder bei reduziertem Druck abdestilliert wird.

5. Verfahren nach Anspruch 1, **dadurch gekennzeichnet, daß** Schritt b) Ammoniak mittels Stripper, Kurzweg- oder Dünnschichtverdampfer abdestilliert wird.

6. Verfahren nach Anspruch 1, **dadurch gekennzeichnet, daß** Schitt c) bei einem pH-Wert zwischen 4 und 6 durchgeführt wird.

7. Verfahren nach Anspruch 1, **dadurch gekennzeichnet, daß** Schritt c) bei einer Temperatur zwischen 0 und 95°C durchgeführt wird.

8. Verfahren nach Anspruch 1, **dadurch gekennzeichnet, daß** in Schritt d) die Zugabe der Fumarsäure bei maximal 100°C, bevorzugt bei maximal 70°C erfolgt.

9. Verfahren nach Anspruch 1, **dadurch gekennzeichnet, daß** das in Schritt f) erhaltene Gemisch aus Ammoniumfumarat und Ammoniak aus Schritt b) mit Ammoniak bis zum Erreichen eines pH-Wertes zwischen 7 und 9 ergänzt und als Edukt in Schritt a) eingesetzt wird.

## Claims

1. Process for preparing L-aspartic acid by enzyme-catalyzed reaction of fumaric acid with ammonia, **characterized in that**
a) fumaric acid is reacted with with ammonia in an inert diluent in the presence of aspartase or aspartase-producing microorganisms to give ammonium L-aspartate, then
b) ammonia is removed from the reaction mixture and
c) L-aspartic acid is precipitated from the remaining reaction mixture by adding formic acid or acetic acid, then the L-aspartic acid is seperated off, washed and dried, and
d) formic acid or acetic acid is released from the mother liquor containing ammonium formate or acetate, respectively, combined with the wash water, by adding fumaric acid,
e) after filtration of the resulting ammonium fumarate solution, the filtrate is used again, with or without fresh formic acid or acetic acid, in step c) to precipitate out L-aspartic acid and
f) the remaining ammonium fumarate is mixed with the ammonia from step b) and this mixture is added as starting material to step a).

2. Process according to Claim 1, **characterized in that** the diluent used in step a) is water, water/ethanol mixture or water/acetone mixture.

3. Process according to Claim 1, **characterized in that** step a) is carried out at a pH between 7 and 9.

4. Process according to Claim 1, **characterized in that**, in step b), distillation is carried out at temperatures between 30 and 110°C and at atmospheric pressure or reduced pressure.

5. Process according to Claim 1, **characterized in that**, in step b), ammonia is distilled off by means of a stripper, short-path evaporator or thin-film evaporator.

6. Process according to Claim 1, **characterized in that** step c) is carried out at a pH between 4 and 6.

7. Process according to Claim 1, **characterized in that** step c) is carried out at a temperature between 0 and 95°C.

8. Process according to Claim 1, **characterized in that**, in step d), fumaric acid is added at a maximum of 100°C, preferably at a maximum of 70°C.

9. Process according to Claim 1, **characterized in that** the mixture obtained in step f) of ammonium formate and ammonia from step b) is supplemented with ammonia until a pH between 7 and 9 is reached and is used as starting material in step a).

## Revendications

1. Procédé de préparation d'acide L-aspartique par réaction, catalysée enzymatiquement, d'acide fumarique avec de l'ammoniac, **caractérisé en ce que**
a) on fait réagir de l'acide fumarique dans un diluant inerte, en présence d'une aspartase ou de microorganismes produisant une aspartase, avec de l'ammoniac pour obtenir du L-aspartate d'ammonium, ensuite
b) on sépare l'ammoniac du mélange de réaction, et
c) on fait précipiter l'acide L-aspartique dans le mélange de réaction restant par addition d'acide formique ou d'acide acétique, ensuite on le sépare, on le lave et on le sèche et
d) on élimine l'acide formique ou l'acide acétique de la solution mère contenant du formiate ou de l'acétate d'ammonium, combinée avec l'eau de lavage, par addition d'acide fumarique,
e) après filtration de la solution ainsi obtenue contenant du fumarate d'ammonium, on réutilise le filtrat, le cas échéant conjointement avec de l'acide formique ou de l'acide acétique frais, dans l'étape c) pour la précipitation de l'acide L-aspartique et
f) on mélange le fumarate d'ammonium restant avec l'ammoniac de l'étape b) et on ajoute ce mélange en tant que produit de départ dans l'étape a).

2. Procédé selon la revendication 1, **caractérisé en ce qu'**on utilise de l'eau, un mélange d'eau/éthanol ou d'eau/acétone en tant que diluant dans l'étape a).

3. Procédé selon la revendication 1, **caractérisé en ce qu'**on effectue l'étape a) à une valeur de pH comprise entre 7 et 9.

4. Procédé selon la revendication 1, **caractérisé en ce que**, dans l'étape b), on effectue la séparation par distillation à des températures comprises entre 30 et 110°C et à la pression normale ou à une pression réduite.

5. Procédé selon la revendication 1, **caractérisé en ce que**, dans l'étape b), on sépare l'ammoniac par distillation au moyen d'extracteurs, d'évaporateurs à trajectoire courte ou en couche mince.

6. Procédé selon la revendication 1, **caractérisé en ce qu'**on effectue l'étape c) à une valeur de pH comprise entre 4 et 6.

7. Procédé selon la revendication 1, **caractérisé en ce qu'**on effectue l'étape c) à une température comprise entre 0 et 95°C.

8. Procédé selon la revendication 1, **caractérisé en ce que**, dans l'étape d), on effectue l'addition de l'acide fumarique à une température maximale de 100°C, de préférence à une température maximale de 70°C.

9. Procédé selon la revendication 1, **caractérisé en ce que**, dans l'étape f), on complète le mélange obtenu de fumarate d'ammonium et d'ammoniac résultant de l'étape b) avec de l'ammoniac jusqu'à l'obtention d'une valeur de pH comprise entre 7 et 9 et on utilise le mélange résultant en tant que produit de départ dans l'étape a).
